# EUROPEAN PATENT APPLICATION

(11) **EP 3 121 706 A1**
(43) Date of publication of application: **25.01.2017**
(21) Application number: 14886323.6
(22) Date of filing: 30.05.2014
(51) Int. Cl.: G06F 3/12

(54) **CONTROL METHOD AND CONTROL SYSTEM FOR WORKING MODE OF MOBILE DEVICE**

(30) Priority: 19.03.2014 CN 201410102151
(71) Applicant: Huizhou TCL Mobile Communication Co., Ltd., Huizhou, Guangdong 516006 (CN)
(72) Inventor: QIAN, Wen, Hui Zhou Guangdong 516006 (CN); ZHENG, Yu, Hui Zhou Guangdong 516006 (CN)
(74) Representative: Brachmann, Roland W.
(86) International application number: PCT/CN2014/078866
(87) International publication number: WO 2015/139371

(57) **Abstract**

The present invention provides a control method and a control system for a working mode of a mobile device. The method comprises: a wearable device obtaining current sign data of a user, and sending the current sign data to a mobile device; the mobile device receiving the sign data, and determining a current motion state of the user according to the sign data; and the mobile device obtaining, according to the current motion state of the user, a working mode corresponding to the current motion state of the user, and switching the current working mode of the mobile device to the corresponding working mode. By means of a wearable device or an accessory device, the state of a user is intelligently determined, and an intelligent terminal is controlled to enter a corresponding working mode. Therefore, like a remote controller, the wearable device realizes a function of intelligently controlling switchover of the working mode, and it is more convenient for the user to use the device.

## Description

### Field of the Invention

The present invention relates to the field of wearable devices and mobile terminals, and in particular relates to a method and a system for controlling operation modes of a mobile device.

### Description of the Related Art

Now that smart phones have increasingly powerful functions and more applications, people are spending more time on cell phones. The power consumption issue has become the biggest issue that smart phone users need to pay attention to. Although cell phone manufacturers have designed many power saving modes, most of the power saving modes designed by manufacturers or developers achieve more power saving from the software level. No solution close to use habits of users has been designed regarding when to turn on or off the power saving mode.

When switching cell phone operation modes, users of smart phones currently available on the market often make manual adjustments according to the environment in which the users are. For example, the meeting mode is selected during a meeting, the silent mode is selected during rest or the outdoor mode is selected during exercises; alternatively, a gravity sensor, an acceleration sensor, a gyroscope or a heartbeat sensor is provided on a smart phone to detect the current state of a user, thereby intelligently adjusting the operation mode of a cell phone. However, no technology that controls the operation mode of an intelligent terminal through other devices has been developed.

Therefore, the prior art is still in need of improvement and development.

### Summary of the Invention

In light of the above drawbacks of the prior art, the object of the present invention is to provide a method and a system for controlling operation modes of a mobile device, which overcomes the drawback that operation modes of mobile devices can only be manually switched or the drawback of intelligent switch based on the state of a mobile device according to the prior art.

The technical solution according to the present invention is as follows:
A method for controlling operation modes of a mobile device, wherein the method comprises steps of:
   A. A wearable device acquires current vital sign data of a user, and transmits the current vital sign data to a mobile device;
   B. The mobile device receives the vital sign data and determines the current motion state of the user according to the vital sign data;
   C. The mobile device acquires an operation mode corresponding to the current motion state of the user according to the current motion state of the user, and switches the current operation mode of the mobile device to the corresponding operation mode.

The method for controlling operation modes of a mobile device, wherein, prior to Step A, it further comprises:
S. The mobile device receives an operation command from the user and sets operation modes corresponding to a plurality of motion states of the user, respectively.

The method for controlling operation modes of a mobile device, wherein Step A specifically comprises:
A1. The wearable device receives an operation command from the user and establishes connection with the mobile device;
A2. The wearable device acquires current vital sign data of the user with a sensor provided therein;
A3. The wearable device receives an operation command from the user and transmits the vital sign data to a mobile device.

The method for controlling operation modes of a mobile device, wherein operation modes of the mobile device comprise a power saving mode, a silent mode and a normal mode.

The method for controlling operation modes of a mobile device, wherein the wearable device establishes connection with the mobile device via any one of Bluetooth, IR, wifi and NFC.

The method for controlling operation modes of a mobile device, wherein the wearable device is a smart watch, smart ring, smart glasses, smart shoes or smart clothing.

A system for controlling operation modes of a mobile device, wherein it comprises:
A monitoring and transmitting module for a wearable device to acquire current vital sign data of a user and to transmit the current vital sign data to a mobile device;
A receiving and determining module for the mobile device to receive the vital sign data and to determine the current motion state of the user according to the vital sign data;
A mode switching module for the mobile device to acquire an operation mode corresponding to the current motion state of the user according to the current motion state of the user, and to switch the current operation mode of the mobile device to the corresponding operation mode.

The system for controlling operation modes of a mobile device, wherein it further comprises:
A setting module for the mobile device to receive an operation command from the user and to set operation modes corresponding to a plurality of motion states of the user, respectively.

The system for controlling operation modes of a mobile device, wherein the monitoring and transmitting module specifically comprises:
A connection unit for the wearable device to receive an operation command from the user and to establish connection with the mobile device;
A state acquiring unit for the wearable device to acquire current vital sign data of the user with a sensor provided therein;
A transmitting unit for the wearable device to receive an operation command from the user and to transmit the vital sign data to a mobile device.

The system for controlling operation modes of a mobile device, wherein the wearable device is a smart watch, smart ring, smart glasses, smart shoes or smart clothing.

The present invention provides a method and a system for controlling operation modes of a mobile device, the method comprising: a wearable device acquires current vital sign data of a user, and transmits the current vital sign data to a mobile device; the mobile device receives the vital sign data and determines the current motion state of the user according to the vital sign data; the mobile device acquires an operation mode corresponding to the current motion state of the user according to the current motion state of the user, and switches the current operation mode of the mobile device to the corresponding operation mode. With the wearable device or accessories thereof, the state of a user is determined intelligently, and a smart terminal is controlled to enter a corresponding operation mode. The wearable device achieves the function of intelligently controlling the operation mode switch like a remote control, making it more convenient for the user to use the device.

### Brief Description of the Drawings

- Fig. 1: is a flow chart of a preferred embodiment of the method for controlling operation modes of a mobile device according to the present invention.
- Fig.2: is a structural block diagram of a preferred embodiment of the system for controlling operation modes of a mobile device according to the present invention.

### Detailed Description of the Embodiments

The present invention provides a method and a system for controlling operation modes of a mobile device. To make the object, technical solution and effect of the present invention clearer and more specific, the present invention will be further described in detail below. It should be understood that the specific embodiments herein are only used to describe the present invention, rather than to limit the present invention.

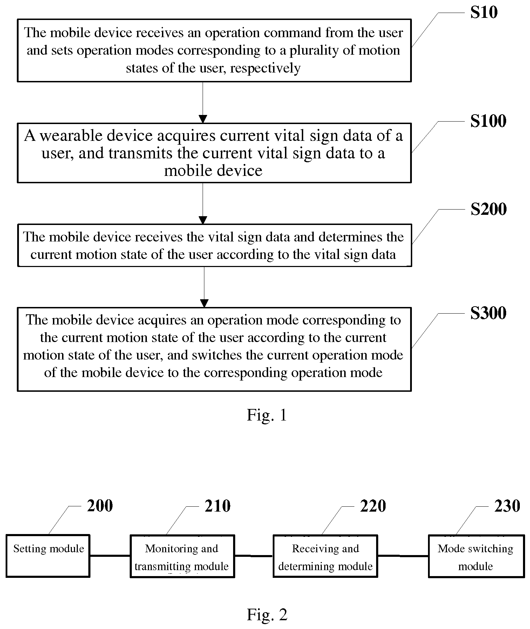

Please refer to Fig.1, and Fig.1 is a flow chart of a preferred embodiment of the method for controlling operation modes of a mobile device according to the present invention. As shown in Fig.1, the method for controlling operation modes of a mobile device comprises the following steps of:
S100. A wearable device acquires current vital sign data of a user, and transmits the current vital sign data to a mobile device;

In S100, the vital sign data of a user are collected in real time through a variety of sensors provided in the wearable device. The vital sign data in the present invention are not, in the strict sense, those data of human organs collected via medical devices in the medical field, but a variety of data of human body in a broader sense collected via a variety of sensors, for example, using a variety of sensors to recognize and record active time and rest time, exercise intensity, number of steps, walking distance and calories burned by a user in an exercise state, recognize and record slight wrist movements in the states of light sleep and deep sleep, record time needed to fall asleep, balance between light sleep and deep sleep, time lying on the bed and times of wake-up during the night, etc. When the wearable device has acquired vital sign data of the user, it transmits the acquired vital sign data to the mobile device.

S200. The mobile device receives the vital sign data and determines the current motion state of the user according to the vital sign data;

In S200, when the mobile device receives the vital sign data, it detects changes to the user behaviors together with the sensors in the mobile device. When the mobile device receives the vital sign data of the user transmitted by the wearable device, it determines the current motion state of the user with reference to data in the human vital sign parameter database stored in the mobile device.

S300. The mobile device acquires an operation mode corresponding to the current motion state of the user according to the current motion state of the user, and switches the current operation mode of the mobile device to the corresponding operation mode.

When the mobile terminal acquires the current motion state of the user, it issues a command to switch the current operation mode of the mobile device. For example, the current heartbeat of a user collected by the wearable device is 75, the data is transmitted to the mobile device. Since a table of correspondence between current state of the user and operation mode of the device is provided in the mobile device, it is learned by comparing with the data in the table that the operation mode of the mobile device corresponding to the current heartbeat is normal mode, then the mobile device is switched to the normal mode; if the current heartbeat of a user collected by the wearable device is 60, the mobile device is switched to the silent mode. The current state of the user is acquired via the wearable device, the mobile device receives the same and then intelligently switches the operation mode based on the data. Even if the mobile device is away from the user at a distance, it can still control remotely the switch of operation modes of the mobile device, leading to great convenience for the user.

In the embodiment, furthermore, as shown in Fig.1, prior to the wearable device establishing connection with the mobile device in S 100, it further comprises:
S10. The mobile device receives an operation command from the user and sets operation modes corresponding to a plurality of motion states of the user, respectively.

The operation modes corresponding to a plurality of motion states of the user, respectively, in S10 in the preferred embodiment of the present invention may be either set by the manufacturer before the mobile device is shipped or set by the user according to the vital sign parameters thereof.

Furthermore, operation modes of the mobile device comprise a power saving mode, a silent mode and a normal mode. Besides the above three modes, more modes may be further set to meet the actual use demand by mobile device users, for example, setting a meeting mode, an exercise mode, etc.

In S100, specific steps of the wearable device establishing connection with the mobile device comprise:
S101. The wearable device receives an operation command from the user and establishes connection with the mobile device;

Wherein the wearable device establishes connection with the mobile device via any one of Bluetooth, IR, wifi and NFC. After the wearable device establishes connection with the mobile device, the wearable device and the mobile device can perform real time data exchange.

S102. The wearable device acquires current vital sign data of the user with a sensor provided therein; wherein the current state of the user comprises a waking state, a light sleep state and a deep sleep state; the determination of the current state of a user is based on a variety of sensors provided in the wearable device, such as acceleration sensor, heartbeat sensor, gyroscope and gravity sensor, that collect parameters of a user, such as the current motion state, heartbeat, pulse, etc., and then the current state of the user is determined in a comprehensive way according to the above parameters.

S103. The wearable device receives an operation command from the user and transmits the vital sign data to the mobile device.

When the wearable device transmits the current vital sign data to the mobile device in S100, the transmission is made regularly or irregularly. Namely, the wearable device automatically transmits the current state data of a user to the mobile device at a fixed interval, or the wearable device irregularly receives a data transmission command from the user and manually transmits the current vital sign data of a user to the mobile device.

In the embodiment, furthermore, the wearable device is a smart watch, smart ring, smart glasses, smart shoes or smart clothing. Smart watch or smart ring is preferred in the preferred embodiment of the present invention, since a user can wear the above two types of wearable devices all day long, and does not need to take it off even during sleep. In such a way, it is favorable for the smart wearable device to collect vital sign parameters of the user in real time, and the mobile device determines to switch to which operation mode based on the data collected by the smart wearable device. The switch of operation modes of the mobile device not only can meet the requirement of the user on the ring volume, but also can meet the requirement of power saving levels.

The present invention will be further described below with reference to a specific application example:
Application example 1: a user enters the rest state or wakes up from the rest state
   101. A smart watch receives a connection establishing command from the user before the user rests, and establishes connection with a smart phone;
   102. The smart watch acquires vital sign parameters of the user during the rest with a variety of sensors provided therein;
   103. The smart watch transmits the collected vital sign parameter data of the user to the smart phone;
   104. Based on the received vital sign parameters of the user, and with reference to the vital sign parameters - current state of the user stored in the smart phone, the smart phone determines whether the user is currently in the sleep state;
   105. When determining that the user is in the sleep state, the mode of the smart phone is adjusted to the silent mode, and at the same time, some applications and functions are restricted;
   106. When determining that the user enters the normal state, the mode of the smart phone is adjusted to the normal mode, and at the same time, the restriction on the above some applications and functions is lifted.

It can be seen that it meets a user's daily life requirements better by switching operation modes of the mobile device through the data exchange between the wearable device and the mobile device, which makes it more convenient for the user to use and control the device.

Based on the above method, the present invention further provides a system for controlling operation modes of a mobile device, as shown in Fig.2, comprising:
A monitoring and transmitting module 210 for a wearable device to acquire current vital sign data of a user and to transmit the current vital sign data to a mobile device; see the above description for details.

A receiving and determining module 220 for the mobile device to receive the vital sign data and to determine the current motion state of the user according to the vital sign data; see the above description for details.

A mode switching module 230 for the mobile device to acquire an operation mode corresponding to the current motion state of the user according to the current motion state of the user, and to switch the current operation mode of the mobile device to the corresponding operation mode; see the above description for details.

In the embodiment, furthermore, as shown in Fig.2, the system for controlling operation modes of a mobile device further comprises:
A setting module 200 for the mobile device to receive an operation command from the user and to set operation modes corresponding to a plurality of motion states of the user, respectively; see the above description for details.

In the embodiment, furthermore, the monitoring and transmitting module 210 specifically comprises:
A connection unit for the wearable device to receive an operation command from the user and to establish connection with the mobile device; see the above description for details.

A state acquiring unit for the wearable device to acquire current vital sign data of the user with a sensor provided therein; see the above description for details.

A transmitting unit for the wearable device to receive an operation command from the user and to transmit the vital sign data to a mobile device; see the above description for details.

In the embodiment, furthermore, the wearable device is a smart watch, smart ring, smart glasses, smart shoes or smart clothing.

In summary, the present invention provides a method and a system for controlling operation modes of a mobile device, the method comprising: a wearable device acquires current vital sign data of a user, and transmits the current vital sign data to a mobile device; the mobile device receives the vital sign data and determines the current motion state of the user according to the vital sign data; the mobile device acquires an operation mode corresponding to the current motion state of the user according to the current motion state of the user, and switches the current operation mode of the mobile device to the corresponding operation mode. With the wearable device or accessories thereof, the state of a user is determined intelligently, and a smart terminal is controlled to enter a corresponding operation mode. The wearable device achieves the function of intelligently controlling the operation mode switch like a remote control, making it more convenient for the user to use the device.

It should be understood that applications of the present invention are not limited to the above examples. To those skilled in the art, improvements or modifications may be made according to the above description, and all of these improvements or modifications shall be encompassed by the appended claims of the present invention.

## Claims

1. A method for controlling operation modes of a mobile device, **characterized in that** the method comprises steps of:
A. A wearable device acquires current vital sign data of a user, and transmits the current vital sign data to a mobile device;
B. The mobile device receives the vital sign data and determines the current motion state of the user according to the vital sign data;
C. The mobile device acquires an operation mode corresponding to the current motion state of the user according to the current motion state of the user, and switches the current operation mode of the mobile device to the corresponding operation mode.

2. The method for controlling operation modes of a mobile device according to Claim 1, **characterized in that**, prior to Step A, it further comprises:
S. The mobile device receives an operation command from the user and sets operation modes corresponding to a plurality of motion states of the user, respectively.

3. The method for controlling operation modes of a mobile device according to Claim 1, **characterized in that** Step A specifically comprises:
A1. The wearable device receives an operation command from the user and establishes connection with the mobile device;
A2. The wearable device acquires current vital sign data of the user with a sensor provided therein;
A3. The wearable device receives an operation command from the user and transmits the vital sign data to a mobile device.

4. The method for controlling operation modes of a mobile device according to Claim 1, **characterized in that** operation modes of the mobile device comprise a power saving mode, a silent mode and a normal mode.

5. The method for controlling operation modes of a mobile device according to Claim 1, **characterized in that** the wearable device establishes connection with the mobile device via any one of Bluetooth, IR, wifi and NFC.

6. The method for controlling operation modes of a mobile device according to any one of Claims 1 to 5, **characterized in that** the wearable device is a smart watch, smart ring, smart glasses, smart shoes or smart clothing.

7. A method for controlling operation modes of a mobile device, **characterized in that** the method comprises steps of:
S. The mobile device receives an operation command from the user and sets operation modes corresponding to a plurality of motion states of the user, respectively;
A. A wearable device acquires current vital sign data of a user, and transmits the current vital sign data to a mobile device;
Step A specifically comprises:
A1. The wearable device receives an operation command from the user and establishes connection with the mobile device;
A2. The wearable device acquires current vital sign data of the user with a sensor provided therein;
A3. The wearable device receives an operation command from the user and transmits the vital sign data to a mobile device;
B. The mobile device receives the vital sign data and determines the current motion state of the user according to the vital sign data;
C. The mobile device acquires an operation mode corresponding to the current motion state of the user according to the current motion state of the user, and switches the current operation mode of the mobile device to the corresponding operation mode.

8. The method for controlling operation modes of a mobile device according to Claim 7, **characterized in that** the vital sign data in Step A are a variety of data of the human body collected via a variety of sensors, including: using a variety of sensors to recognize and record active time and rest time, exercise intensity, number of steps, walking distance and calories burned by a user in an exercise state; recognizing and recording slight wrist movements in the states of light sleep and deep sleep, recording time needed to fall asleep, balance between light sleep and deep sleep, time lying on the bed and times of wake-up during the night.

9. The method for controlling operation modes of a mobile device according to Claim 7, **characterized in that** operation modes of the mobile device comprise a power saving mode, a silent mode and a normal mode.

10. The method for controlling operation modes of a mobile device according to Claim 7, **characterized in that** the wearable device establishes connection with the mobile device via any one of Bluetooth, IR, wifi and NFC.

11. The method for controlling operation modes of a mobile device according to any one of Claims 7 to 10, **characterized in that** the wearable device is a smart watch, smart ring, smart glasses, smart shoes or smart clothing.

12. A method for controlling operation modes of a mobile device, **characterized in that** the method comprises steps of:
S. The mobile device receives an operation command from the user and sets operation modes corresponding to a plurality of motion states of the user, respectively;
A. A wearable device acquires current vital sign data of a user, and transmits the current vital sign data to a mobile device;
B. The mobile device receives the vital sign data and determines the current motion state of the user according to the vital sign data;
C. The mobile device acquires an operation mode corresponding to the current motion state of the user according to the current motion state of the user, and switches the current operation mode of the mobile device to the corresponding operation mode;
The vital sign data in Step A are a variety of data of the human body collected via a variety of sensors, including: using a variety of sensors to recognize and record active time and rest time, exercise intensity, number of steps, walking distance and calories burned by a user in an exercise state; recognizing and recording slight wrist movements in the states of light sleep and deep sleep, recording time needed to fall asleep, balance between light sleep and deep sleep, time lying on the bed and times of wake-up during the night.

13. The method for controlling operation modes of a mobile device according to Claim 12, **characterized in that** Step A specifically comprises:
A1. The wearable device receives an operation command from the user and establishes connection with the mobile device;
A2. The wearable device acquires current vital sign data of the user with a sensor provided therein;
A3. The wearable device receives an operation command from the user and transmits the vital sign data to a mobile device.

14. A system for controlling operation modes of a mobile device, **characterized in that** it comprises:
- A monitoring and transmitting module for a wearable device to acquire current vital sign data of a user and to transmit the current vital sign data to a mobile device;
- A receiving and determining module for the mobile device to receive the vital sign data and to determine the current motion state of the user according to the vital sign data;
- A mode switching module for the mobile device to acquire an operation mode corresponding to the current motion state of the user according to the current motion state of the user, and to switch the current operation mode of the mobile device to the corresponding operation mode.

15. The system for controlling operation modes of a mobile device according to Claim 14, **characterized in that** it further comprises:
- A setting module for the mobile device to receive an operation command from the user and to set operation modes corresponding to a plurality of motion states of the user, respectively.

16. The system for controlling operation modes of a mobile device according to Claim 14, **characterized in that** the monitoring and transmitting module specifically comprises:
- A connection unit for the wearable device to receive an operation command from the user and to establish connection with the mobile device;
- A state acquiring unit for the wearable device to acquire current vital sign data of the user with a sensor provided therein;
- A transmitting unit for the wearable device to receive an operation command from the user and to transmit the vital sign data to a mobile device.

17. The system for controlling operation modes of a mobile device according to any one of Claims 14 to 16, **characterized in that** the wearable device is a smart watch, smart ring, smart glasses, smart shoes or smart clothing.
